Europäisches Patentamt

(19) European Patent Office

Office européen des brevets

(11) Publication number: **0 362 360 B1**

## (12) EUROPEAN PATENT SPECIFICATION

(45) Date of publication of patent specification: **29.12.93**   (51) Int. Cl.⁵: **C02F   1/46**

(21) Application number: **89904677.5**

(22) Date of filing: **23.03.89**

(86) International application number:
**PCT/US89/01201**

(87) International publication number:
**WO 89/09190 (05.10.89 89/24)**

(54) **A STERILIZED AEROSOL CONTAINER HAVING AN AOUEOUS SALINE SOLUTION THEREIN AND THE METHOD FOR STERILIZING THE CONTAINER.**

(30) Priority: **24.03.88 US 172646**

(43) Date of publication of application:
**11.04.90 Bulletin 90/15**

(45) Publication of the grant of the patent:
**29.12.93 Bulletin 93/52**

(84) Designated Contracting States:
**AT BE CH DE FR GB IT LI LU NL SE**

(56) References cited:
| | |
|---|---|
| GB-A- 2 094 992 | US-A- 2 061 323 |
| US-A- 2 440 915 | US-A- 3 443 055 |
| US-A- 3 479 275 | US-A- 3 547 600 |
| US-A- 3 616 355 | US-A- 3 763 900 |
| US-A- 4 761 208 | US-A- 4 822 472 |

(73) Proprietor: **Abplanalp, Robert Henry
10 Hewitt Avenue
Bronxville New York 10708(US)**

(72) Inventor: **Abplanalp, Robert Henry
10 Hewitt Avenue
Bronxville New York 10708(US)**

(74) Representative: **Knoblauch, Ulrich, Dr.-Ing. et al
Kühhornshofweg 10
D-60320 Frankfurt (DE)**

EP 0 362 360 B1

## Description

The invention relates to a method for sterilizing an aerosol container having a saline solution having a bactericidal/bacteriostatic activity as a component therein; the resulting package comprising an aerosol valved pressure container including the saline solution; and in a further aspect, a method for creating a saline solution having a bactericidal or bacteriostatic activity for dispensing onto a surface to be sterilized.

Isotonic saline solutions are used, for example, in the washing and cleaning of "soft" contact lenses. The isotonic saline solution, in one form, is packed into an aerosol container with a propellant. The solution is then dispensed onto the contact lens when the lens needs cleaning. It is essential that the aerosol saline product be free of bacteria.

To insure freedom from bacteria, the industry presently packs the saline solution and propellant into a sealed aerosol container having a manually actuatable valve disposed in the container opening. After the aerosol unit is formed, the entire unit is then transported to a radiation facility and the entire unit is radiated. This operation is costly.

Moreover, subsequent to the radiation treatment, the saline solution of the aerosol unit does not possess residual bacteria killing or bacteria controlling activity.

The lack of bactericidal or bacteriostatic activity in an isotonic saline solution that has been sterilized by radiation creates the problem that this solution cannot sterilize "soft" contact lenses. "Soft" lenses, which normally consist of a copolymer of mainly a hydrophilic monomer, such as a 2-hydroxy ethyl methacrylate, have approximately thirty percent (30%) by weight of water, or more, and thus the lenses themselves offer a vehicle or environment suitable for the propagation of various kinds of bacteria. The use of bacteria contaminated contact lenses may cause serious injury to the human oculus tissue.

Therefore, as a method for sterilizing a water-containing contact lens ("soft" lens), there has been proposed to boil the lens for a predetermined period of time. Although boiling is very effective for sterilization, it has the following defects:

(1) The protein in lachrymal liquid adhered to the lens due to boiling comes to fix the surface of the lens as it is thermally degenerated, which not only spoils the optical property of the lens but also makes the lens feel very uncomfortable when in place in the eye.

(2) The slightly cross-linked hydrophilic copolymer of 2-hydroxyethyl methacrylate material for the water-containing contact lens deteriorates by the repeated boiling treatments so that the lens becomes discolored or the rated configuration thereof changes to greatly shorten the life of the lens.

(3) Due to the use of an AC power source for a boiling heater, it is inconvenient to carry the sterilizer in travel and especially in an outdoor invironment such as a camp site or the like, the sterilizer cannot be used, because no power source is available there.

(4) Non-water-containing contact lenses made of polyethyl methacrylate or silicon rubber are not suitable for boiling sterilization.

As a means for overcoming such difficulties involved in the boiling sterilization, there has been proposed a method for sterilizing a contact lens with various kinds of sterilizing liquid medicines like thimerosal or chlorohexydine. However, such method has the drawback that due to the molecular spacing in the structure of the water-containing contact lens, the sterilizing component in the sterilizing liquid medicine tends to be absorbed into the lens and cause an anaphylactic inflammation of the oculus tissue.

Beside the above method, there is also a prior method in which the lens is sterilized with a 3% sodium hydrogen peroxide solution, and a catalyst such as platinum is brought into contact with the solution to decompose the sodium hydrogen peroxide into water and oxygen so that the lens is made sterile. However, this method has the drawbacks that a long time is required for sterilization and the process is complicated and impractical.

Accordingly, an object of the present invention is to provide a method for sterilizing an aerosol container including a saline solution having a bactericidal/bacteriostatic activity, in particular and without limitation, an isotonic saline solution; and the resultant sterilized saline solution in an aerosol container, which method and product avoid the drawbacks of the prior art.

In accordance with the invention there are provided a package as defined in claim 1 and methods as defined in claims 4 and 8.

### Detailed Description of the Invention

The invention comprises an efficient method for sterilizing a sealed container having an aqueous saline solution having a bactericidal/bacteriostatic activity as at least one component thereof. In one form, a saline solution is electrolyzed in an electrolytic cell and sealed in an aerosol container with a valve-bearing

2

mounting cup and then pressure filled with propellant. In a more specific form of this aspect, the saline solution is an isotonic saline solution.

In another aspect the invention comprises a sealed pressurized saline solution having bactericidal and/or bacteriostatic activity, said active saline solution being generated by subjecting the saline solution to electrolysis. In the preferred form, the saline solution, more preferably, an isotonic saline solution is passed through an electrolytic cell immediately prior to entering the pressurized (aerosol) container, the container is then closed with a closure (mounting cup) bearing a manually actuatable valve , and the container pressure filled with propellant.

The invention will be hereafter described with reference to an isotonic saline solution (0.9% NaCl).

An electrolytic cell useful in sterilizing the isotonic saline solution is described in US-A-3,443,055, the disclosure of which being incorporated into and made a part of the subject disclosure. Also incorporated into the subject disclosure is US-A-3,616,355 showing a method of generating enhanced biocidal activity in the electrolysis of chloride containing solutions at higher voltages than those disclosed in the '055 patent.

Other electrolytic cells suitable for sterilizing the isotonic saline solution are described in US-A-3,479,275 and US-A-3,547,600.

In packaging the isotonic saline solution in an aerosol container, and in the preferred form, the saline solution is flowed through a suitable electrolytic cell and into a contiguous aerosol container and the container sealed by crimping a closure (mounting cup) bearing a manually-actuatable valve onto the open end or bead of the container. The container is then pressure filled with propellant.

It has been found that flowing an isotonic saline solution through electrode plates under the following conditions and parameters produced a product having the capability of sterilizing the saline solution and the inner surfaces of an aerosol container and other components in the container. These containers further produced a saline solution having bactericidal/bacterio-static properties over a protracted period. The conditions and parameters are:

One litre (1 l) of an isotonic saline solution was passed into an electrolytic cell wherein the electrodes were spaced 6.86 mm (.270") apart. The facing surfaces of the electrodes were 76.2 mm by 50.8 mm (3" by 2"). An electric power of 7.8 volts and 5.0 amps was applied to the electrolytic cell and the saline solution released from the cell at the average time of 35 seconds. The samples discussed hereafter had two passes through the electrolytic cell unless otherwise noted.

The experimental data supporting the aforesaid statement regarding bactericidal/bacteriostatic activity are set forth hereafter.

Solutions of sodium chloride in distilled water (isotonic saline solution) were prepared and treated in several different ways before being packaged in an aerosol container fitted with an aerosol valve.

Several different microorganisms were used in this study and each strain of microorganism was added to a different container of saline solution. Some of the solutions were then passed through an electrolytic cell and packaged into aerosol containers. Samples from each can were sprayed immediately after filling into the aerosol container and plated onto agar media. The plates were observed for bacterial growth after 24, 48 and 72 hours. Stored samples were also sprayed onto additional agar media and observed according to the above time schedule. Additionally, samples which were originally contaminated with microorganisms and found to be sterile after passing through the electrolytic cell were re-contaminated with microorganisms after standing about three (3) weeks. These were then sprayed as above and according to the same schedule.

The results obtained to date indicate the following:

1. An isotonic saline solution will support the growth of microorganisms.

2. An isotonic saline solution packaged in an aerosol container will maintain sterility if the product is initially sterile.

3. An isotonic saline solution containing microorganisms can be sterilized by passing the solution through an electrolytic cell.

4. An isotonic saline solution passed through an electrolytic cell, and added to an aerosol can containing microorganisms, is capable of killing the microorganisms and rendering the solution, can, valve and nitrogen propellant sterile.

5. After about three (3) weeks microorganisms were added to an aerosol can filled with a sterile saline solution which had previously been passed through the electrolytic cell. The microorganisms were killed within a 24 hour period.

Experimental

I. Preparation of Saline Solutions (0.9% by weight)

A. Formulation

All solutions were prepared by taking 9.0 grams of Sodium Chloride, U.S.P. (United States Pharmacopoeia) and adding it to sufficient distilled water to make 1 l of solution. This is what is referred to as isotonic saline solution and is the type of solution used in the non-preserved contact lens cleaning solutions. Saline solutions prepared in this manner were used in this study.

B. Packaging

All aerosols were prepared by adding 200 ml of the saline solution (treated or non-treated) to an aluminium aerosol container (sold by the company Peerless Tube as 53,06 mm (2.089") X 165 mm (6 1/2") #203 internally lined) and sealing the can with a valve (sold by the Precision Valve Company as 0,457 mm (0.018") Stem X 0.457 mm (0,018") Body). Nitrogen gas was then added to the filled container at a pressure of 6.90 bar (100 psig). A suitable actuator was then added to the valve.

C. Microorganisms Used

The following microorganisms were used in this study:
1. Escherichia Coli - is a gram-negative non-spore forming bacillus. It is found as normal flora in the intestinal tract. Some pathogenic strains of E. Coli have the ability to invade the intestinal mucosa and produce enterotoxins. The symptoms seen are fever, chills, abdominal pain and dysentery.
  - The bacteria grows very well on MacConkey agar and nutrient agar (pink colonies observed).
  - The bacteria grows best at an incubation 35-37°C.
  - The bacteria grows well in a saline environment.
  - One could observe growth within a 24-48 hour period.
2. Pseudomonas Aeruginosa - is a gram-negative obligate aerobe rod. It is commonly found in soil, water, sewage and (in a small percentage) in the intestinal tract.
  - The bacteria grows well on MacConkey agar and nutrient agar. The organism is known to produce a bluish pigment named pyocyanin and a greenish fluorescent pigment known as fluorescein.
  - The bacteria grows best at an incubation of 35-37°C and grows well in a saline environment. Growth was observed within a 48-72 hour period.
These organisms are opportunistic, infecting individuals who are debilitated, burned or immunosuppressed.
3. Salmonella - This organism is commonly found in spoiled foods and is responsible for food poisoning.

D. Media Used

Two types of media were used to determine if bacterial growth was taking place. When no growth was noted, then the product was deemed to be sterile. Petri dishes containing the appropriate growth media were sprayed with the saline solution which had been treated in different ways. The following types of media were used:
A. MacConkey Agar was used to enhance the growth of gram-negative bacteria.
B. Columbia CNA Agar was used to enhance the growth of gram-positive bacteria.

II. Results

The following combinations of saline solution, microorganisms and treatment through the electrolytic cell were studied to date:
A. Non-treated Saline Solution (Control)
B. Non-treated Saline Solution + Microorganism
C. Saline Solution + Electrolytic Cell
D. Saline Solution + Microorganism + Electrolytic Cell
E. Saline Solution + Electrolytic Cell + Aerosol Can Contaminated with Microorganisms
Tables I and.II indicate the results obtained to date.

4

TABLE I

Sterility of Saline Solution (0.9%)

| Treatment | Organism Added | | Electro-lytic Cell | MacConkey Media | CNA Media | Microbial Growth* 37°C Time | | | |
|---|---|---|---|---|---|---|---|---|---|
| | To Solution | To Can | | | | 24 Hrs | 48 Hrs | 72 Hrs | Repeat |
| Distilled Water | --- | --- | No | Yes | Yes | NG | NG | NG | NG |
| Saline Solution (Non-Aerosol) | --- | --- | No | Yes | Yes | NG | NG | NG | NG |
| Saline Solution (Non-Aerosol) | --- | --- | Yes | Yes | Yes | NG | NG | NG | NG |
| Saline Solution (Aerosol) | --- | --- | No | Yes | Yes | NG | NG | NG | NG |
| Saline Solution (Aerosol) | --- | --- | Yes | Yes | Yes | NG | NG | NG | NG |

NG = No Growth

* Even though no growth was noted, this is not an indication that growth will not occur if organisms are present. It just happened that no organisms were present in these samples.

EP 0 362 360 B1

TABLE II

Sterility of Saline Solution (0.9%) - With Microorganisms Added

| Treatment | Organism Added To Solution | To Can | Electro-lytic Cell | MacConkey Media | CNA Media | Microbial Growth 37°C Time | | | |
|---|---|---|---|---|---|---|---|---|---|
| | | | | | | 24 Hrs | 48 Hrs | 72 Hrs | Repeat after 3 weeks |
| Saline Solution (Aerosol) | E. Coli | --- | No | Yes | Yes | +++ | +++ | +++ | +++ |
| Saline Solution (Aerosol) | E. Coli | --- | Yes | Yes | Yes | NG | NG | NG | NG |
| Saline Solution (Aerosol) | --- | E. Coli | No | Yes | Yes | +++ | +++ | +++ | +++ |
| Saline Solution (Aerosol) | --- | E. Coli | Yes | Yes | Yes | NG | NG | NG | NG |
| Saline Solution (Aerosol) | Pseudomonas | --- | No | Yes | Yes | +++ | +++ | +++ | +++ |
| Saline Solution (Aerosol) | Pseudomonas | --- | Yes | Yes | Yes | NG | NG | NG | NG |
| Saline Solution (Aerosol) | --- | Pseudomonas | No | Yes | Yes | +++ | +++ | +++ | +++ |
| Saline Solution (Aerosol) | --- | Pseudomonas | Yes | Yes | Yes | NG | NG | NG | NG |
| Saline Solution (Aerosol) | Salmonella | --- | No | Yes | Yes | +++ | +++ | +++ | +++ |
| Saline Solution (Aerosol) | Salmonella | --- | Yes | Yes | Yes | NG | NG | NG | NG |
| Saline Solution (Aerosol) | --- | Salmonella | No | Yes | Yes | +++ | +++ | +++ | +++ |
| Saline Solution (Aerosol) | --- | Salmonella | Yes | Yes | Yes | NG | NG | NG | NG |

+++ = Abundant Growth
NG = No Growth

Streptococcus F alkalies were also studied in a manner similar to the other organisms. Samples of organism were added to isotonic saline solution packages in an aerosol can. The samples packaged without passing through the electrolytic cell showed a great amount of bacterial growth while the samples passed through the cell and then packaged in an aerosol container showed no signs of growth.

6

The bactericidal/bacteriostatic properties of the isotonic saline solution is believed to be attributable to the formation of a combination of ozone, hydrogen peroxide and sodium hypochlorite.

In the past, passing saline solutions through an electrolytic cell has produced a solution having a short time span of sterilization capability. However, it was unexpected to discover that a saline solution passed through an electrolytic cell and promptly packaged in a pressurized vessel, such as an aerosol caontainer, would retain the sterilization capability over a protracted period. Samples prepared according to the above have been found to retain their sterilization capability for a period approaching four months.

Chemical and Physical Studies

Drop in NaCl Contents After Electrolysis

| | $AgNO_3$ (ml) | NaCl (mg) | $AgNO_3$ (ml) | NaCl (mg) | $AgNO_3$ (ml) | NaCl (mg) |
|---|---|---|---|---|---|---|
| Not Treated | 15.62 15.60 | 9.1225 | 15.60 15.61 | 9.1196 | 15.70 15.60 | 9.1450 |
| Passed Through Cell | 15.40 15.50 | 9.0290 | 15.35 15.35 | 8.9705 | 15.30 15.26 | 8.9296 |
| 2nd Day | 15.40 15.40 | 8.9998 | 15.30 15.30 | 9.2050 | 15.22 15.24 | 8.9004 |
| 6th Day | 15.35 15.30 | 8.9559 | 15.30 15.25 | 8.9267 | 15.20 15.20 | 8.8829 |
| 12th Day | 15.35 15.37 | 8.9764 | 15.23 15.27 | 8.9121 | 15.20 15.22 | 8.8887 |
| 18th Day | 15.37 15.37 | 8.9822 | 15.21 15.26 | 8.9033 | 15.20 15.20 | 8.8829 |
| 24th Day | 15.36 15.36 | 8.9822 | 15.24 15.26 | 8.9121 | 15.20 15.20 | 8.8829 |

These results were obtained by titration with $AgNO_3$.

The drop in sodium chloride content is consistent with the formation of sodium hypochlorite. The sterilization capability is affected by the duration of time that the saline solution is subjected to electrolysis; also suggesting that an increase in voltage may also affect the sterilization capability. This is demonstrated by the following data:

| | Can #1 | Can #2 | Average |
|---|---|---|---|
| Before Cell | 9.0465 | 9.0407 | 9.0436 |
| After 1 Pass | 8.9413 | 8.9413 | 8.9413 |
| 2 Passes | 8.8829 | 8.8946 | 8.8887 |
| 3 Passes | 8.8595 | 8.8712 | 8.8654 |
| 4 Passes | 8.8244 | 8.8303 | 8.8274 |
| 6 Passes | 8.7660 | 8.7660 | 8.7660 |
| 10 Passes | 8.6900 | 8.6783 | 8.6842 |

While the method of the invention has been described in terms of "electrolyzing" the saline solution outside the aerosol container, it should be understood that the pressure (aerosol) container, per se, may function as an electrolytic cell by the appropriate disposition of electrodes and electrical connection as part of the container.

Further, the method of the invention has been specifically described with reference to nitrogen as the propellant. Other propellants, both liquified gas and compressed gas may be utilized. In this connection, the method has been described in terms of adding propellant through pressure filling nitrogen gas. However, if other than compressed gases are employed as the propellant, other systems, such as, for example, under-the-cup filling of propellant may be employed.

Additionally, while the invention has been illustrated by describing an aerosol container wherein the propellant is disposed in the container in contact with the saline solution, it should be understood (and it is presently viewed by the inventor to be the best form for an aerosol container having sterilized saline solution) that the saline solution may be packed in an aerosol container of the so-called "piston" type. In the "piston" type aerosol container, the product to be dispensed and the propellant are separated by a piston which moves along the longitudinal axis of the container in response to the pressure generated by the propellant on one side of the piston. Actuation of the valve on the product side of the piston provides an opening for the product to egress from the container. Closing the valve terminates flow of product and movement of piston.

## Claims

1. A package comprising an aerosol valved pressure container having an aqueous electrolyzed sterilizing saline solution of sodium chloride having bactericidal/bacteriostatic activity under Superatmospheric pressure stored therein.

2. The package of claim 1, wherein the saline solution is an isotonic saline solution.

3. A package comprising a valved pressure container having an aqueous electrolyzed saline solution of sodium chloride under Superatmospheric pressure stored therein, said saline solution having a bactericidal/bacteriostatic activity lasting at least about 120 days from the time of electrolysis.

4. A method for creating a solution to be dispensed having a long-term bactericidal/bacteriostatic activity comprising sealing in an aerosol container under superatmospheric pressure a saline solution of sodium chloride having bactericidal/bacteriostatic activity that has been subjected to the forces of an electrolytic cell immediately prior to entering the pressurized container.

5. The method of claim 4, wherein the saline solution is an isotonic saline solution.

6. The method of claim 4 or 5, wherein the superatmospheric pressure is generated by disposing the saline solution in contact with a pressure generating propellant.

7. The method of claim 4 or 5, wherein the superatmospheric pressure is generated by disposing the saline solution on the valved side of a piston disposed within the aerosol container and by disposing a pressure generating propellant on the nonvalved side of the piston.

8. A method for sterilizing an aerosol container comprising sealing in the container under superatmospheric pressure a saline solution of sodium chloride that has been subjected to the forces of an electrolytic cell immediately prior to entering the pressurized container, said saline solution having bactericidal/bacteriostatic activity lasting at least about 120 days from the time of electrolysis.

## Patentansprüche

1. Packung mit einem ein Ventil aufweisenden Aerosol-Druckbehälter, der eine wässrige elektrolysierte sterilisierende Salzlösung aus Natriumchlorid mit bakterizider/bakteriostatischer Aktivität unter Überatmosphärendruck enthält.

2. Packung nach Anspruch 1, bei der die Salzlösung eine isotonische Salzlösung ist.

3. Packung mit einem ein Ventil aufweisenden Aerosol-Druckbehälter, der eine wässrige elektrolysierte Salzlösung aus Natriumchlorid unter Überatmosphärendruck enthält, die eine bakterizide/bakteriostatische Aktivität aufweist, die wenigstens etwa 120 Tage vom Zeitpunkt der Elektrolyse an andauert.

4. Verfahren zum Erzeugen einer auszugebenden Lösung mit einer langzeitigen bakteriziden/bakteriostatischen Aktivität, bei dem in einem Aerosolbehälter unter Überatmosphärendruck eine Salzlösung aus Natriumchlorid mit bakterizider/bakteriostatischer Aktivität dicht abgeschlos-

EP 0 362 360 B1

sen ist und den Kräften einer elektrolytischen Zelle unmittelbar vor dem Einfüllen in den Druckbehälter ausgesetzt wurde.

5. Verfahren nach Anspruch 4, bei dem die Salzlösung eine isotonische Salzlösung ist.

6. Verfahren nach Anspruch 4 oder 5, bei dem der Überatmosphärendruck dadurch erzeugt wird, daß die Salzlösung mit einem Druckerzeugungs-Treibmittel in Berührung gebracht wird.

7. Verfahren nach Anspruch 4 oder 5, bei dem der Überatmosphärendruck dadurch erzeugt wird, daß die Salzlösung auf der mit einem Ventil versehenen Seite eines Kolbens angeordnet wird, der in dem Aerosolbehälter angeordnet ist, und daß ein Druckerzeugungs-Treibmittel auf der nicht mit einem Ventil versehenen Seite des Kolbens angeordnet wird.

8. Verfahren zum Sterilisieren eines Aerosolbehälters, bei dem eine Salzlösung aus Natriumchlorid in dem Behälter unter Überatmosphärendruck dicht abgeschlossen wird, die unmittelbar vor dem Einfüllen in den Druckbehälter den Kräften einer elektrolytischen Zelle ausgesetzt wurde und eine bakterizide/bakteriostatische Aktivität aufweist, die wenigstens etwa 120 Tage vom Zeitpunkt der Elektrolyse an andauert.

**Revendications**

1. Emballage comprenant un conteneur aérosol à pression muni d'une valve, renfermant, sous une pression supérieure à la pression atmosphérique, une solution saline aqueuse de chlorure de sodium électrolysée stérilisante ayant une activité bactéricide/bactériostatique.

2. Emballage selon la revendication 1, dans lequel la solution saline est une solution saline isotonique.

3. Emballage comprenant un conteneur à pression muni d'une valve, renfermant, sous une pression supérieure à la pression atmosphérique, une solution saline aqueuse de chlorure de sodium électrolysée, cette solution saline ayant une activité bactéricide/bactériostatique d'une durée d'au moins environ 120 jours à partir du moment de l'électrolyse.

4. Procédé pour préparer une solution destinée à être distribuée, ayant une activité bactéricide/bactériostatique à long terme consistant à sceller dans un conteneur pour aérosol, sous une pression supérieure à la pression atmosphérique, une solution saline de chlorure de sodium ayant une activité bactéricide/bactériostatique qui a été soumise aux forces d'une cellule électrolytique aussitôt avant de pénétrer dans le conteneur pressurisé.

5. Procédé selon la revendication 4, dans lequel la solution saline est une solution saline isotonique.

6. Procédé selon la revendication 4 ou 5, dans lequel la pression supérieure à la pression atmosphérique est produite en mettant la solution saline en contact avec un propulseur produisant une pression.

7. Procédé selon la revendication 4 ou 5, dans lequel la pression supérieure à la pression atmosphérique est produite en disposant la solution saline du côté muni d'une valve d'un piston disposé à l'intérieur du conteneur aérosol et en disposant un propulseur produisant une pression du côté non muni d'une valve du piston.

8. Procédé pour stériliser un conteneur aérosol consistant à sceller dans le conteneur, sous une pression supérieure à la pression atmosphérique, une solution saline de chlorure de sodium qui a été soumise aux forces d'une cellule électrolytique aussitôt avant de pénétrer dans le conteneur sous pression, cette solution saline ayant une activité bactéricide/bactériostatique d'une durée d'au moins 120 jours à partir du moment de l'électrolyse.

9